# EUROPEAN PATENT APPLICATION

(11) **EP 4 458 299 A1**
(43) Date of publication of application: **06.11.2024**
(21) Application number: 22914670.9
(22) Date of filing: 26.12.2022
(51) Int. Cl.: A61B 18/12, A61B 18/14

(54) **ABLATION APPARATUS**

(30) Priority: 31.12.2021 CN 202123451073 U
(71) Applicant: Shenzhen Lifetech Respiration Scientific Co., Ltd., Shenzhen, Guangdong 518057 (CN)
(72) Inventor: HU, Longhu, Shenzhen, Guangdong 518057 (CN); SHI, Yue, Shenzhen, Guangdong 518057 (CN); LI, Anning, Shenzhen, Guangdong 518057 (CN)
(74) Representative: Prinz & Partner mbB
(86) International application number: PCT/CN2022/141951
(87) International publication number: WO 2023/125416

(57) **Abstract**

An ablation device is provided, including a main body structure (110), electrodes (130), and a plurality of supporting arms (120), where the supporting arm (120) is inclined outward relative to the main body structure (110) and includes a contact portion (121); the contact portion (121) has an arcuate structure, and a convex surface of the arcuate structure faces a side of the supporting arm (120) away from a center of the main body structure (110); the electrode (130) is mounted on the contact portion (121) and covers at least a part of an outer side wall of the contact portion (121). Thus, when the supporting arm (120) is squeezed by an air tube (300), the contact portion (121) forms an arcuate structure, and the convex surface of the arcuate structure faces the side of the supporting arm (120) away from the main body structure (110), causing the contact portions (121) on different supporting arms (120) to at least partially attach to a gap (340) between cartilages (330) of the air tube (300). The electrode (130) is fixed on the contact portion (121) and covers at least a part of the outer wall of the contact portion (121), causing the electrodes (130) on different supporting arms (120) to at least partially attach to the gap (340) between the cartilages (330) of the air tube (300). In this way, the ablation device forms a circumferential continuous ablation energy field on an inner wall of the air tube (300), thus avoiding ablation loss caused by a part of the electrodes (130) not attaching to the inner wall of the air tube (300).

## Description

### Technical Field

The present disclosure relates to the field of medical devices, and particularly to an ablation device.

### Background Art

This section merely provides background information related to the present disclosure and is not necessarily related art.

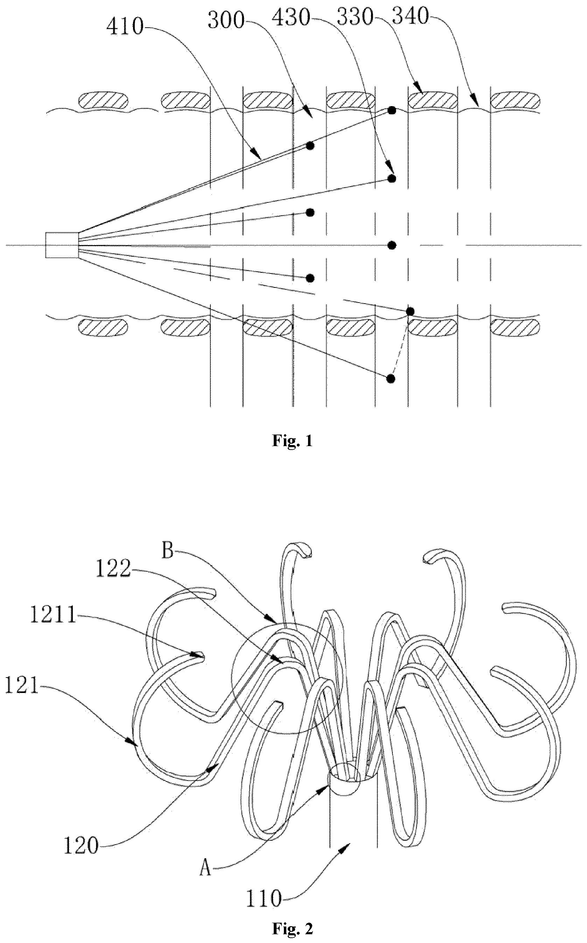

In the related art, as shown in Fig. 1 (the dotted line part in Fig. 1 is a schematic diagram of an electrode supporting arm 410 sliding under the pressure of an air tube 300, and the solid line part is a schematic structural diagram of the electrode supporting arm 410 in a natural state), in order to better adapt the ablation device to a D-shaped profile of the air tube 300, the electrode supporting arms 410 are generally provided as a divergent structure. An electrode 430 is coupled to an end portion of the electrode supporting arm 410, and when the divergent electrode supporting arms 410 are squeezed, pressures received by different electrode supporting arms 410 are different, causing displacement of the end portions of a part of the electrode supporting arms 410 so that the electrode 430 slides from a gap 340 between cartilages 330 to the cartilage 330. It is difficult for the electrode 430 to ablate the nerve outside the cartilage 330, thereby affecting the ablation effect on the air tube 300.

### Summary of the Invention

Based on this, it is necessary to provide an ablation device, including a main body structure, electrodes, and a plurality of supporting arms, where the supporting arm is inclined outward relative to the main body structure and includes a contact portion; the contact portion has an arcuate structure, and a convex surface of the arcuate structure faces a side of the supporting arm away from a center of the main body structure; the electrode is arranged on the contact portion and covers at least a part of an outer side wall of the contact portion.

Optionally, an end of the contact portion away from the body structure is provided toward an inner side of the body structure.

Optionally, the supporting arm includes a first connecting arm and a second connecting arm connected, the first connecting arm is connected to the contact portion through the second connecting wall, and the first connecting arm and the second connecting arm are provided at a predetermined included angle.

Optionally, the ablation device further includes an elastic constraining structure; the elastic constraining structure is at least connected to two supporting arms and is configured to constrain the amount of movement of the supporting arms connected to the elastic constraining structure when subjected to pressure.

Optionally, the elastic constraining structure includes a first constraining member and a second constraining member connected, and the first constraining member and the second constraining member are provided at a predetermined included angle; the first constraining member is connected to one of the supporting arms, and the second constraining member is connected to the other of the supporting arms.

Optionally, the ablation device further includes an adjustment assembly; the adjustment assembly is connected to all of the supporting arms and is configured to take all of the supporting arms to move toward a side close to the center of the main body structure or toward a side away from the center of the main body structure.

Optionally, the adjustment assembly includes a pulling member and a plurality of connecting members, and all of the connecting members are provided along a circumference of the pulling member at intervals; one end of the connecting member is connected to the pulling member, and the other end of the connecting member is connected to one of the supporting arms.

Optionally, the connecting member includes a first bent portion and a second bent portion that are connected; the first bent portion is bent from a side of the connecting member away from the main body structure to a side close to the main body structure, and the second bent portion is bent from the side of the connecting member close to the main body structure to the side away from the main body structure.

Optionally, a guide hole is arranged on the main body structure and is arranged along an axial direction of the main body structure, and the pulling member is inserted into the guide hole and slides in the guide hole.

Optionally, the electrode includes a strip electrode.

Compared with the related art, the ablation device of the present disclosure has the following beneficial effects.

When the supporting arm is squeezed by an air tube, the contact portion forms an arcuate structure, and the convex surface of the arcuate structure faces the side of the supporting arm away from the main body structure, causing the contact portions on different supporting arms to at least partially attach to a gap between cartilages of the air tube. The electrode is fixed on the contact portion and covers at least a part of the outer wall of the contact portion, causing the electrodes on different supporting arms to at least partially attach to the gap between the cartilages of the air tube. In this way, the ablation device forms a circumferential continuous ablation energy field on an inner wall of the air tube, thus avoiding ablation loss caused by a part of the electrodes not attaching to the inner wall of the air tube.

### Brief Description of the Drawings

Fig. 1 is a schematic structural diagram of a divergent electrode supporting arm releasing in an air tube according to the related art;
Fig. 2 is a schematic structural diagram of an ablation device according to embodiment 1 of the present disclosure;
Fig. 3 is an enlarged schematic diagram of a structure at A in Fig. 2 according to the present disclosure;
Fig. 4 is a schematic structural diagram of a cross-section of an air tube in the related art;
Fig. 5 is a schematic structural diagram of an ablation device releasing in an air tube according to embodiment 1 of the present disclosure;
Fig. 6 is a schematic structural diagram showing the mounting of an electrode on a supporting body according to embodiment 1 of the present disclosure;
Fig. 7 is an enlarged schematic diagram of a structure at B in Fig. 2 according to the present disclosure;
Fig. 8 is a schematic structural diagram of an ablation device according to embodiment 2 of the present disclosure;
Fig. 9 is an enlarged schematic diagram of a structure at C in Fig. 8 according to the present disclosure;
Fig. 10 is a schematic structural diagram showing the connecting of an adjustment assembly and a supporting body according to the present disclosure;
Fig. 11 is a schematic structural diagram of an adjustment assembly according to the present disclosure; and
Fig. 12 is an enlarged schematic diagram of a structure at D in Fig. 11 according to the present disclosure.

### Detailed Description of the Invention

In order to make the above-mentioned objects, features, and advantages of the present invention more obvious and understandable, the specific implementations of the present invention are described in detail below in conjunction with the accompanying drawings. Many specific details are set forth in the following description to facilitate a full understanding of the present invention. However, the present invention can be implemented in many other ways different from those described herein, and a person skilled in the art may make similar improvements without contravening the connotation of the present invention, and therefore the present invention is not subject to the specific implementations disclosed below.

Unless otherwise defined, all technical and scientific terms used herein have the same meanings as generally understood by a person skilled in the art of the present invention. The terms used in the description of the present invention herein are for the purpose of describing specific embodiments only and are not intended to limit the present invention.

### Embodiment 1

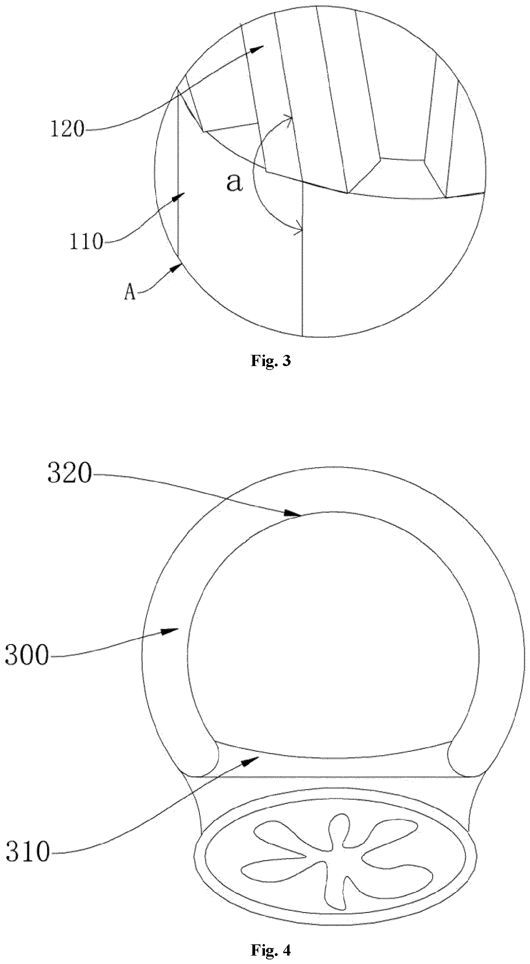

Embodiments of the present disclosure provide an ablation device, as shown in Figs. 2 and 3, including a main body structure 110, electrodes, and a plurality of supporting arms 120. All of the supporting arms 120 are spaced apart along a circumference of the main body structure 110. The supporting arm 120 is inclined outward relative to the main body structure 110 and includes a contact portion 121. The contact portion 121 is of an arcuate structure, and a convex surface of the arcuate structure faces a side of the supporting arm 120 away from the center of the main body structure 110. The electrode is arranged on the contact portion 121 and covers at least a part of an outer side wall of the contact portion 121.

The ablation device is configured to deliver ablation energy at the tissue to be ablated in the human body, and the ablation energy can ablate nerve tissue, thereby achieving the effect of treating a lesion site in the human body. The ablation device can be used for ablation of the air tube, heart, aorta, stomach, etc. For example, in this embodiment, the ablation device is delivered into the air tube through a delivery device, and then the ablation device ablates nerve tissues of the air tube, thereby expanding the air tube and achieving treatment of a pulmonary disease.

The main body structure 110 provides a supporting force for the supporting arm 120 and can be a tubular structure, a cylindrical structure, or any other structure that provides support for the supporting arm 120. The main body structure 110 can be formed by cutting metal material or by weaving woven wires.

As shown in Figs. 2 and 3, the supporting arm 120 can be made of memory metal material or elastic material and has a certain elastic recovery force after being bent or deformed. The supporting arm 120 is welded or integrally connected to the main body structure 110 and is inclined relative to an axial direction of the main body structure 110. Specifically, there define an included angle "a" between a side wall of the supporting arm 120 and an axis of the main body structure 110. The included angle "a" is 90° to 175°, for example, the included angle "a" can be 90°, 110°, 120°, 135°, 160°, or 175°. A plurality of supporting arms 120 are provided. The plurality of supporting arms 120 are provided along the circumference of the main body structure 110 at spaced interval. Two adjacent supporting arms 120 have a spacing distance, and the spacing distance can be equal or different. All of the supporting arms 120 define a divergent structure.

As shown in Fig. 4, the cross-section of an air tube 300 has a D-shaped profile, and the air tube 300 includes a smooth muscle 310 and a C-shaped profile 320. After the ablation device is released in the air tube 300, the supporting arm 120 attaching to the smooth muscle 310 is subjected to a large squeezing force by the air tube 300, thereby causing a large deformation amount of the part of the supporting arm 120 after the force is applied, and the supporting arm 120 attaching to the C-shaped profile is subjected to a small squeezing force by the air tube 300, thereby causing a small deformation amount of the part of the supporting arm 120 after the force is applied.

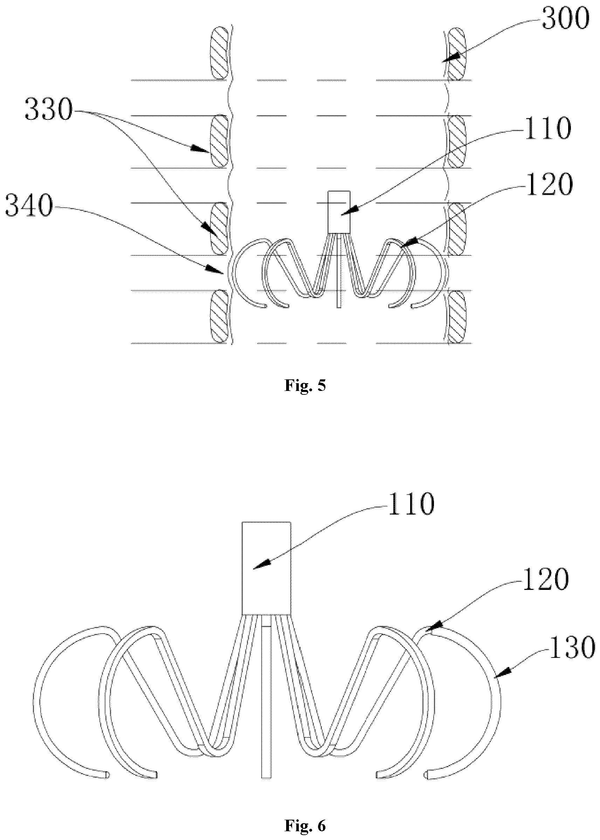

As shown in Fig. 2, the supporting arm 120 includes the contact portion 121. The contact portion 121 is located at an end of the supporting arm 120 away from the main body structure 110 and has an arcuate structure, and the convex surface of the arcuate structure faces the side of the supporting arm 120 away from the center of the main body structure 110. As shown in Fig. 5, the air tube 300 includes a plurality of cartilages 330 provided along an axial direction of the air tube300 at spaced intervals, and when the ablation device is released in the air tube 300, the contact portion 121 attaches to a gap 340 between two adjacent cartilages 330.

As shown in Fig. 6, an electrode 130 is fixed on the contact portion 121 and covers at least a part of the outer wall of the contact portion 121, and the electrode 130 outputs radio frequency energy to destroy nerves in the tissue of the air tube 300 after being energized. The electrode 130 can be connected to the contact portion 121 by welding, bonding, encapsulation, hot-melting, and other processes, and the material of the electrode 130 includes a metal material such as titanium, silver, gold, platinum alloy, palladium, and alloy. The number of the electrode 130 is not limited in this embodiment and can be specifically determined according to practical application. The electrode 130 includes a strip electrode, a dot electrode, or a mesh electrode. In other embodiments, the contact portion 121 is made of conductive material to form an electrode.

Since the supporting arm 120 is inclined relative to the main body structure 110, allowing all of the supporting arms 120 to define a divergent structure on the whole. As shown in Fig. 1, when the supporting arms 120 are subjected to the squeezing force from the air tube 300, the deformation degree between two ends of different supporting arms 120 along the axial direction of the main body structure 110 are different. For example, the supporting arm 120 attaching to the smooth muscle 310 is subjected to a large pressure, and accordingly, the deformation degree of the supporting arm 120 is also greater, resulting in a greater relative distance between the two ends of the supporting arm 120 in the axial direction of the body structure 110. The supporting arm 120 attaching to the C-shaped profile 320 is subjected to a small pressure, and accordingly, the deformation degree of the supporting arm 120 is also less, resulting in a lesser relative distance between the two ends of the supporting arm 120 in the axial direction of the main body structure 110. Therefore, after the supporting arms 120 are released in the air tube 300, the supporting arms 120 are subjected to the squeezing force from the air tube 300, and one end of a part of the supporting arms 120 can cover the gap 340 and attach to the cartilage 330, making it difficult for the electrode 430 to ablate the nerve outside the cartilage 330, affecting the ablation effect of the ablation device on the air tube 300.

Thus, when the supporting arm 120 is squeezed by the air tube 300, the contact portion 121 forms an arcuate structure, and the convex surface of the arcuate structure faces the side of the supporting arm away from the main body structure 110, causing the contact portions 121 on different supporting arms to at least partially attach to the gap between the cartilages of the air tube 300. The electrode 130 is fixed on the contact portion 121 and covers at least a part of the outer wall of the contact portion 121, causing the electrodes 130 on different supporting arms to at least partially attach to the gap between the cartilages of the air tube 300. In this way, the ablation device form a circumferential continuous ablation energy field on an inner wall of the air tube 300, thus avoiding ablation loss caused by a part of the electrodes 130 not attaching to the inner wall of the air tube 300.

Further, in order to prevent an end of the contact portion 121 away from the main body structure 110 from rubbing with the air tube 300 to damage the air tube 300 during deformation of the supporting arm 120 under compression, as shown in Fig. 2, the end of the contact portion 121 away from the main body structure 110 is provided toward a side of the supporting arm 120 close to the center of the main body structure 110. The contact portion 121 includes a first end 1211. The first end 1211 is an end of the contact portion 121 pointing at the axial direction of the main body structure 110. In this embodiment, the first end 1211 is oriented parallel to a radial direction of the main body structure 110. When the contact portion 121 is squeezed by the inner wall of the air tube 300, the first end 1211 moves toward the inner side of the air tube 300, thereby preventing the first end 1211 from scratching the inner wall of the air tube 300. In other embodiments, the first end 1211 can also extend toward an inner side of the main body structure 110 so that a centering angle of the contact portion 121 is greater than 180°.

Further, the first end 1211 is covered with a flexible layer. The flexible layer can be made of flexible material with a certain elasticity, such as rubber, silicone, and PEBAX, and it can decrease the frictional force between the first end 1211 and the inner wall of the air tube, thereby preventing the first end 1211 from scratching the inner wall of the air tube. In other embodiments, an end portion of the first end 1211 can exhibit a spherical or arcuate shape so that the frictional force between the first end 1211 and the inner wall of the air tube can be slowed, thereby preventing the first end 1211 from scratching the inner wall of the air tube.

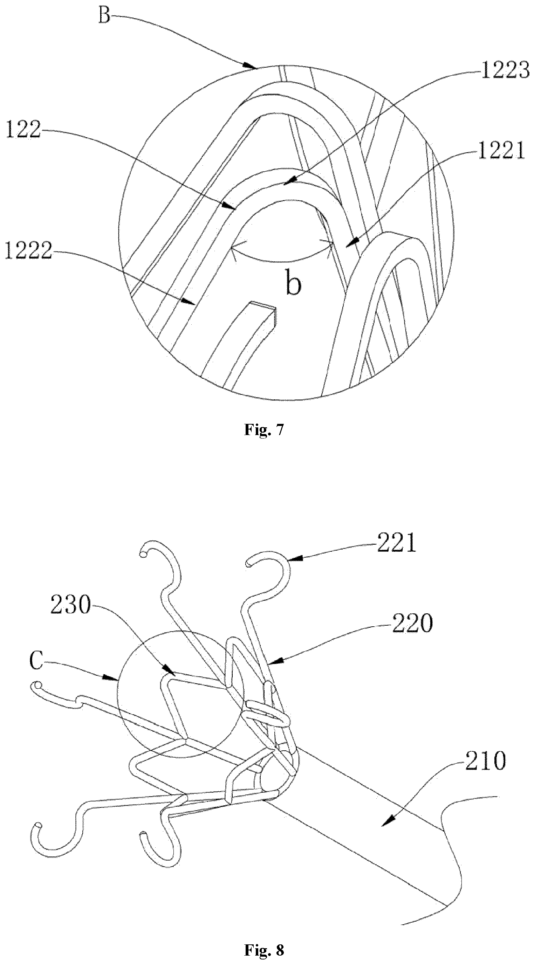

Further, in order to facilitate the movement of the contact portion 121 toward the inner side of the air tube 300 when being subjected to the pressure of the inner wall of the air tube 300, as shown in Figs. 2 and 7, the supporting arm 120 includes a bent portion 122.

One end of the bent portion 122 is connected to the main body structure 110, and the other end of the bent portion 122 is connected to the contact portion 121.

In this embodiment, the bent portion 122 includes a first connecting arm 1221 and a second connecting arm 1222, one end of the first connecting arm 1221 is connected to the main body structure 110, and one end of the second connecting arm 1222 is connected to the contact portion 121. There is an included angle b between the first connecting arm 1221 and the second connecting arm 1222. The included angle "b" ranges from 20°-70°, and specifically, can be 20°, 30°, 45°, 50°, 60°, or 70°. When the contact portion 121 is subjected to the pressure of the inner wall of the air tube 300, the contact portion 121 pushes one end of the second connecting arm 1222 to move toward the first connecting arm 1221.

Thus, when the contact portion 121 is subjected to the pressure of the air tube 300, one end of the bent portion 122 is connected to the body structure 110, and the other end of the bent portion 122 is connected to the contact portion 121 so that the bent portion 122 provide a space for the movement of the contact portion 121 forwarding to the inner side of the air tube 300, and thus the supporting arm 120 adapt to air tubes 300 with different inner diameters.

Further, in order to reduce the resistance of one end of the first connecting arm 1221 to move toward the second connecting arm 1222, as shown in Fig. 7, an arcuate segment 1223 is provided at a position where the first connecting arm 1221 and the second connecting arm 1222 are connected, and the arcuate segment 1223 is configured to reduce the resistance of the second connecting arm 1222 to bend toward the first connecting arm 1221.

Further, during ablation, in order to cool the electrode and the inner wall of the air tube 300, interiors of the main body structure 110 and the supporting arm 120 are communicated to form a cooling channel. In the embodiment, the main body structure 110 and the supporting arm 120 both have a cavity structure, and the cooling channel is arranged in the interior of the main body structure 110 and the supporting arm 120. A cooling medium circulates in the cooling channel and takes away heat from the inner wall of the air tube 300 and the electrode to cool the electrode and the inner wall of the air tube 300.

### Embodiment 2

As shown in Fig. 8, the embodiment differs from embodiment 1 in that the ablation device further includes an elastic constraining structure 230. The elastic constraining structure 230 is connected to two circumferentially adjacent supporting arms 220, and when the supporting arms 220 are squeezed by the air tube, the elastic constraining structure 230 is configured to constrain the amount of displacement of the two adjacent supporting arms 220 to generate relative movement.

It is worth explaining that the ablation device includes a loaded state and an expanded state. When the ablation device needs to be delivered into the air tube 300, the supporting arm 220 and a main body structure 210 are compressed to be loaded into the delivery device, and the delivery device delivers the ablation device into the air tube 300, at which time the ablation device is in the loaded state; when the ablation device need to be released in the air tube 300, the delivery device releases the ablation device in the air tube 300 to cause ablation of the air tube 300, at which time the ablation device is in the expanded state. Since the supporting arm 220 includes a contact portion 221, when a plurality of the supporting arms 220 are squeezed to be loaded into the delivery device, the contact portions 221 easily catch on each other, resulting in a difficulty in releasing the supporting arms 220 in the air tube 300.

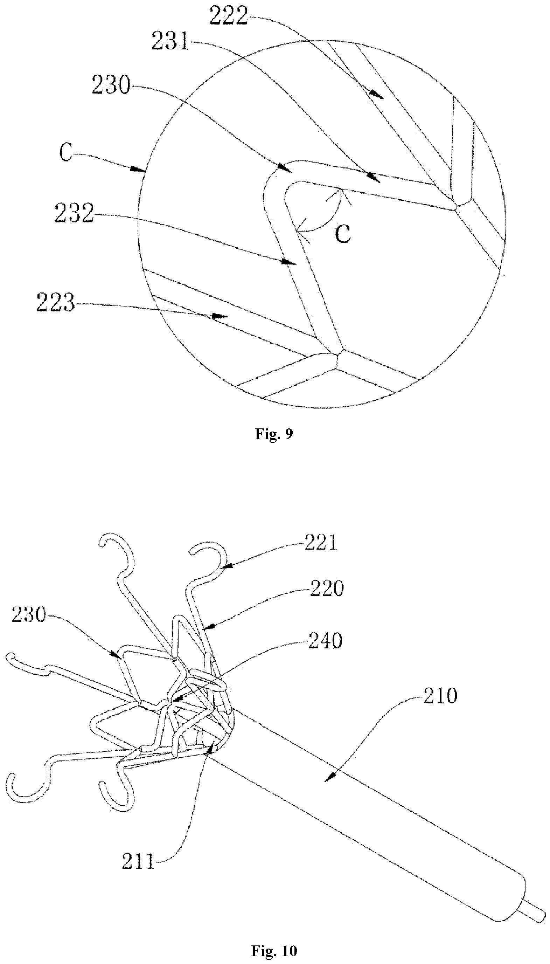

In this embodiment, as shown in Fig. 9, the elastic constraining structure 230 includes a first constraining member 231 and a second constraining member 232 connected to the first constraining member 231, and the first constraining member 231 and the second constraining member 232 are provided at a predetermined included angle. The first constraining member 231 is connected to one of the supporting arms 220, and the second constraining member 232 is connected to the other of the supporting arms 220. The first constraining member 231 and the second constraining member 232 are made of elastic material or memory metal material. Specifically, the first constraining member 231 and the second constraining member 232 are integrally connected, and two circumferentially adjacent supporting arms 220 include a first supporting arm 222 and a second supporting arm 223. One end of the first constraining member 231 is connected to the first supporting arm 222, and one end of the second constraining member 232 is connected to the second supporting arm 223. There is an included angle "c" between the first constraining member 231 and the second constraining member 232. The angle of the included angle "c" ranges from 20°-70°, and specifically, can be 20°, 30°, 45°, 50°, 60°, or 70°. In one embodiment, when the first constraining member 231 and the second constraining member 232 are squeezed by the supporting arms 220, the first constraining member 231 and the second constraining member 232 move toward each other; when the squeezing force is eliminated, the first constraining member 231 and the second constraining member 232 push the supporting arms 220 to reset. Thus, by providing the first constraining member 231 and the second constraining member 232 at a predetermined included angle, there is a certain movement space between the first constraining member 231 and the second constraining member 232 so that there is still a sufficient movement space between two axially adjacent supporting arms 220, thereby facilitating loading of the ablation device into the delivery device.

In other embodiments, the elastic constraining structure 230 includes a spring-shaped elastic member, and two ends of the elastic member are connected to two circumferentially adjacent supporting arms 220, respectively. When the elastic member is squeezed by the two adjacent supporting arms 220, the elastic member contracts and provides an elastic supporting force for the supporting arms 220, and when the squeezing of the elastic member by the supporting arms 220 is eliminated, the supporting arms 220 are reset under the elastic force of the elastic member.

The advantage of this arrangement is that when the supporting arms 220 are squeezed by the delivery device, the elastic constraining structure 230 is connected to the two circumferentially adjacent supporting arms 220 so that the elastic constraining structure 230 provide an elastic supporting force for the supporting arms 220. When the two circumferentially adjacent supporting arms 220 move toward each other to a certain extent, the elastic supporting force of the elastic constraining structure 230 on the supporting arms 220 is greater than the squeezing force of the supporting arms 220 on the elastic constraining structure 230 so that the two circumferentially adjacent supporting arms 220 always maintain a certain spacing distance, thereby preventing the contact portions 221 on two circumferentially adjacent supporting arms 220 from catching on each other, and reducing the difficulty of releasing the supporting arms 220 in the air tube 300.

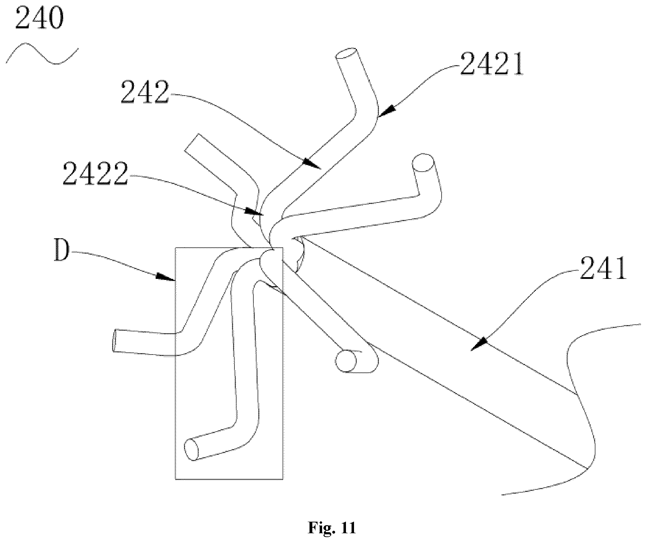

Further, in order to facilitate adjusting an opening and closing degree (namely, the included angle a between the supporting arm 220 and the body structure 210) of the supporting arm 220, as shown in Figs. 10 and 11, the ablation device further includes an adjustment assembly 240. The adjustment assembly 240 is connected to all of the supporting arms 220 and is configured to take all of the supporting arms 220 to move toward a side close to the center of the body structure 210 or toward a side away from the center of the body structure 210.

It is worth explaining that since there is a certain deviation between a position before the ablation device is released and a target position, when the supporting arms 220 are released, it is difficult for the contact portion 221 to directly attach to the target position. At this time, it is necessary to push the supporting arms 220 to make the contact portion 221 attach to the target position, and in order to ensure the attachment of the contact portion 221, there is a certain pressure between the contact portion 221 and the air tube 300, which makes it difficult for the staff to directly push the supporting arms 220 to slide in the air tube 300.

In this embodiment, as shown in Fig. 11, the adjustment assembly 240 includes a pulling member 241 and a plurality of connecting members 242, and all of the connecting members 242 are provided along a circumference of the pulling member 241. One end of the connecting member 242 is connected to the pulling member 241, and the other end of the connecting member 242 is connected to the supporting arm 220.

The pulling member 241 is made of flexible material or rigid material. The pulling member 241 is provided along an axial direction of the main body structure 210 and can be provided in parallel with the main body structure 210 or located inside the main body structure 210. The pulling member 241 can move along the axial direction of the main body structure 210.

The connecting member 242 is made of flexible material or elastic material. One end of the connecting member 242 is integrally connected or rotatably connected to the pulling member 241, and the other end of the connecting member 242 is integrally connected or rotatably connected to the supporting arm 220.

All of the connecting members 242 are provided along the circumference of the pulling member 241 at spaced intervals, and each connecting member 242 is connected to one or more supporting arms 220.

In one embodiment, when it is necessary to reduce the opening and closing degree (namely, the included angle "a" between the supporting arm 220 and the main body structure 210) of the supporting arm 220, the pulling member 241 is pulled to move toward a side away from the supporting arm 220, and the pulling member 241 takes the supporting arm 220 to move toward the side close to the center of the main body structure 210 through the connecting member 242, thereby reducing the opening and closing degree of the supporting arm 220; when it is necessary to reset the supporting arm 220, the pulling member 241 is released to eliminate the constraint on the supporting arm 220 by the connecting member 242, and the supporting arm 220 moves toward the side away from the center of the main body structure 210 under the elastic force, thereby achieving the resetting of the supporting arm 220; when it is necessary to increase the opening and closing degree of the supporting arm 220, the pulling member 241 is pushed to move toward a side close to the supporting arm 220, and the pulling member 241 pushes the supporting arm 220 to move toward the side away from the center of the main body structure 210 through the connecting member 242, thereby increasing the opening and closing degree of the supporting arm 220.

In other embodiments, the adjustment assembly 240 includes a plurality of pull lines, one end of each pull line is connected to the supporting arm 220, and the pull line can move along the axial direction of the main body structure 210. When it is necessary to reduce the opening and closing degree of the supporting arm 220, the pull line is pulled to make the supporting arm 220 move toward the side close to the center of the main body structure 210. When it is necessary to reset the supporting arm 220, the pull line is released to eliminate the constraint on the supporting arm 220, and the supporting arm 220 moves toward the side away from the center of the main body structure 210 under the elastic force, thereby achieving the resetting of the supporting arm 220.

The advantage of this arrangement is that by connecting the adjustment assembly 240 to all of the supporting arms 220, the adjustment assembly 240 can take all of the supporting arms 220 to move toward the side close to the center of the main body structure 210 or toward the side away from the center of the main body structure 210, thereby achieving adjustment of the opening and closing degrees of the supporting arms 220. When it is necessary to adjust the position of the contact portion in the air tube 300, the opening and closing degree of the supporting arm 220 is reduced to reduce an attachment force between the supporting arm 220 and the inner wall of the air tube 300, thereby facilitating adjustment of the position of the contact portion in the air tube 300.

Further, in order to keep the opening and closing degrees of all of the supporting arms 220 consistent during the movement of the supporting arms 220 driven by the pulling member 241, as shown in Fig. 10, a guide hole 211 is arranged on the main body structure 210 and is arranged along the axial direction of the main body structure 210, and the pulling member 241 is inserted into the guide hole 211 and slides in the guide hole 211.

The axis line of the guide hole 211 coincides with an axis line of the main body structure 210. The inner wall of the guide hole 211 is attached to an outer wall of the pulling member 241, and the guide hole 211 extends through two axial ends of the main body structure 210 along the axial direction of the main body structure 210. The pulling member 241 is inserted into the guide hole 211, and one end of the pulling member 241 away from the connecting member 242 extends out of the guide hole 211. Thus, the pulling member 241 is inserted into the guide hole 211 so that the guide hole 211 can define the pulling member 241 at the center of the main body structure 210 to prevent the pulling member 241 from tilting during the movement and causing different forces on different connecting members 242, thereby making the forces on all of the connecting members 242 uniform, and keeping the opening and closing degrees of all of the supporting arms 220 consistent during the movement of the supporting arms 220 driven by the pulling member 241.

Further, in order to facilitate the pulling member 241 to take the supporting arm 220 to move toward the side close to the center of the main body structure 210, as shown in Fig. 11, the connecting member 242 includes a first bent portion 2421 and a second bent portion 2422. The first bent portion 2421 is bent from a side of the connecting member 242 away from the main body structure 210 to a side close to the main body structure 210, and the second bent portion 2422 is bent from the side of the connecting member 242 close to the main body structure 210 to the side away from the main body structure 210.

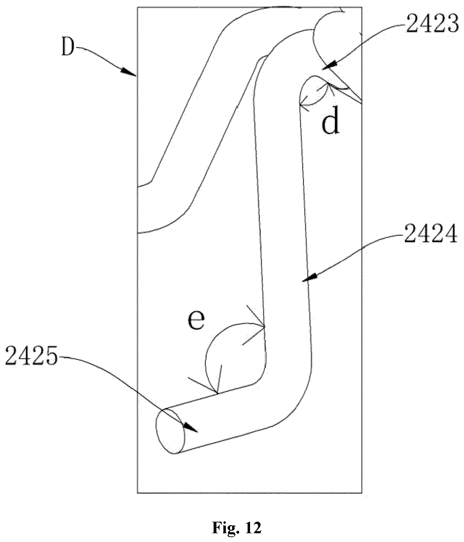

In this embodiment, as shown in Fig. 12, the connecting member 242 includes a third connecting arm 2423, a fourth connecting arm 2424, and a fifth connecting arm 2425 connected sequentially. The two ends of the fourth connecting arm 2424 are connected to the third connecting arm 2423 and the fifth connecting arm 2425, respectively. The third connecting arm 2423 is connected to the pulling member 241, and the fifth connecting arm 2425 is connected to the supporting arm 220. There is an included angle between the third connecting arm 2423 and the fourth connecting arm 2424, and an opening of the included angle "d" is arranged toward the main body structure 210 to form the first bent portion 2421. The angle of the included angle "d" ranges from 10°-35°, and specifically, is 10°, 15°, 20°, 30° and 35°. There is an included angle "e" between the fourth connecting arm 2424 and the fifth connecting arm 2425, and the opening of the included angle "e" is arranged toward the side away from the main body structure 210 to form the second bent portion 2422. The angle of the included angle "e" ranges from 15-90°, and specifically, is 15°, 30°, 45°, 60°, 70°, or 90°. Thus, by including the first bent portion 2421 and the second bent portion 2422 in the connecting member 242, the connecting member 242 is more easily bent and deformed, thereby reducing the difficulty that the pulling member 241 drives the supporting arm 220 to move through the connecting member 242.

The technical features of the above-mentioned embodiments can be arbitrarily combined, and in order to make the description concise, not all the possible combinations of the technical features in the above-mentioned embodiments are described. However, as long as there is no contradiction between the combinations of these technical features, they should be considered as the scope of the description.

The above-mentioned embodiments express only several embodiments of the present invention, which are described in a specific and detailed manner, but are not to be construed as limiting the patent scope of the present invention. It should be noted that several variations and modifications can be made by a person skilled in the art without departing from the conception of the present invention, all of which fall within the scope of the present invention. Therefore, the scope of the present invention patent shall be subject to the attached claims.

## Claims

1. An ablation device, comprising a main body structure, electrodes, and a plurality of supporting arms, wherein the supporting arm is inclined outward relative to the main body structure and comprises a contact portion; the contact portion forms an arcuate structure, and a convex surface of the arcuate structure faces a side of the supporting arm away from a center of the main body structure; the electrode is coupled to the contact portion and covers at least a part of an outer side wall of the contact portion.

2. The ablation device according to claim 1, wherein an end of the contact portion away from the main body structure is provided toward an inner side of the main body structure.

3. The ablation device according to claim 1, wherein the supporting arm comprises a first connecting arm and a second connecting arm, the first connecting arm is connected to the contact portion through the second connecting arm, and the first connecting arm and the second connecting arm are provided at a predetermined included angle.

4. The ablation device according to claim 1, further comprising an elastic constraining structure, wherein the elastic constraining structure is connected to two supporting arms and is configured to constrain the amount of movement of the supporting arms.

5. The ablation device according to claim 4, wherein the elastic constraining structure comprises a first constraining member and a second constraining member, the first constraining member is connected with the second constraining member, and a predetermined included angle is provided between the first constraining member and the second constraining member; the first constraining member is connected to one of the supporting arms, and the second constraining member is connected to the other of the supporting arms.

6. The ablation device according to claim 1, further comprising an adjustment assembly, wherein the adjustment assembly is connected to all of the supporting arms and is configured to take all of the supporting arms to move toward a side close to the center of the main body structure or toward a side away from the center of the main body structure.

7. The ablation device according to claim 6, wherein the adjustment assembly comprises a pulling member and a plurality of connecting members, and all of the connecting members are provided along a circumference of the pulling member at spaced intervals; one end of the connecting member is connected to the pulling member, and the other end of the connecting member is connected to one of the supporting arms.

8. The ablation device according to claim 7, wherein the connecting member comprises a first bent portion and a second bent portion; the first bent portion is bent from a side of the connecting member away from the main body structure to a side close to the main body structure, and the second bent portion is bent from the side of the connecting member close to the main body structure to the side away from the main body structure.

9. The ablation device according to claim 7, wherein a guide hole is arranged on the main body structure and is arranged along the axial direction of the main body structure, and the pulling member is inserted into the guide hole and slides in the guide hole.

10. The ablation device according to claim 1, wherein the electrode comprises a strip electrode.
